# EUROPÄISCHE PATENTANMELDUNG

(11) **EP 0 585 826 A1**
(43) Veröffentlichungstag der Anmeldung: **09.03.1994**
(21) Anmeldenummer: 93113702.0
(22) Anmeldetag: 26.08.1993
(51) Int. Cl.: A61B 17/34

(54) **Medizinisches Instrument zur Schaffung eines Gewebekanals**

(30) Priorität: 02.09.1992 DE 4229310
(71) Anmelder: OLYMPUS WINTER & IBE GmbH, D-22045 Hamburg (DE)
(72) Erfinder: Buess, Gerhard, Prof. Dr. med., D-72074 Tübingen (DE); Melzer, Andreas, D-65199 Wiesbaden (DE); Löffler, Monika, D-22143 Hamburg (DE); Wiegand, Michael, D-21509 Glinde (DE)
(74) Vertreter: Schaefer, Konrad, Dipl.-Phys.

(57) **Zusammenfassung**

Ein medizinisches Instrument zur Schaffung eines zur Aufnahme einer Hülse bestimmten Gewebekanales, mit einem Schaftrohr (11), in dessen distalem Endbereich eine Messerklinge (12) angeordnet ist, die zur Erzeugung einer Schnittfläche ausgebildet ist, sowie mit einer Einrichtung zur Umformung der Schnittfläche zum Gewebekanal, ist dadurch gekennzeichnet, daß die Messerklinge (12) durch einen in Achsrichtung überstehenden Teilbereich der distalen Rohrkante des Schaftrohres (11) gebildet ist und mit einem in Umfangsrichtung des Schaftrohres (11) schneidenden Schliff versehen ist, und daß ein in dem Schaftrohr (11) anordenbarer Einsatz (21) vorgesehen ist, dessen distales Ende (24) stumpf ausgebildet ist und sich in etwa auf Höhe des distalen Endes des Schaftrohres (11) befindet.

## Beschreibung

Die Erfindung bezieht sich auf ein Instrument nach dem Oberbegriff des Anspruches 1.

Gattungsgemäße Instrumente werden im Zuge laparoskopischer, z.B. in der Bauchhöhle vorgenommener Eingriffe eingesetzt.

Bei derartigen Eingriffen wird zunächst die Bauchdecke in einem geeigneten Bereich mit einer dünnen Kanüle durchstochen und der Bauchraum über die Kanüle mit Gas insuffliert.

Dann werden oberhalb des Eingriffsziels eine oder mehrere Öffnungen in der Bauchdecke geschaffen. In diese Öffnungen werden sogenannte Trokarhülsen eingesetzt. Durch diese mit einem Gasventil versehenen Trokarhülsen können während des Eingriffes die erforderlichen laparoskopischen Instrumente in die Bauchhöhle eingeführt werden.

Um eine gute Gasdichtigkeit zu gewährleisten, ist es erforderlich, daß die in der Bauchdecke geschaffenen Öffnungen einen über ihre Länge möglichst konstanten Querschnitt aufweisen. Derartige Öffnungen erlauben eine paßgenaue und damit gasdichte Aufnahme der Trokarhülsen.

Zur Herstellung der Öffnungen werden in der Regel sogenannte Trokare verwendet, die an ihrem Ende einen Dreikantschliff besitzen. Diese Trokare werden durch die Bauchdecke gestoßen und die damit geschaffene Öffnung gegebenenfalls mit Hilfe von Dilatatoren aufgeweitet.

Nachteilig ist jedoch, daß der Operateur zum Durchstoßen der Bauchdecke eine relativ große Kraft auf das Trokar ausüben muß. Eine genaue Kontrolle des Einstichvorganges bzw. eine feine Dosierung des Vorschubes ist nicht möglich. Insbesondere, wenn der Operateur als Einstichstelle einen Bereich der Bauchdecke wählt, der möglicherweise mit Organen verwachsen ist, kann dies fatale Folgen haben.

Einige Operateure ziehen es daher vor, die Bauchdecke im Wege der sogenannten "Minilaparoskopie" zu öffnen. Hierbei dringt man mit einem Skalpell mit kurzen, vorsichtig geführten Schnitten in die Bauchdecke ein. Die Technik erfordert lediglich geringe Kräfte, hat jedoch den Nachteil, daß die hergestellte Öffnung relativ undefiniert und damit möglicherweise nicht zur z. B. abdichtenden Aufnahme einer Trokarhülse geeignet ist.

Es wird auf die DE 34 03 962 C2 verwiesen, aus der ein gattungsgemäßes Instrument zur Schaffung eines Gewebekanales bekannt ist, das einen Schaft aufweist, in dessen distalen Endbereich ein in Achsrichtung schneidendes Messer angeordnet ist. Bei Vorschieben des Schaftes durch das Gewebe wird eine plane Schnittfläche konstanter Breite über die gesamte Vorschublänge erzeugt, die ohne Kraftaufwand durch seitliche Verdrängung der an die Schnittfläche angrenzenden Gewebebereiche zu dem gewünschten Kanal aufgeweitet werden kann. Auch bei diesem Instrument stellt sich jedoch die Frage, ob der hergestellte Kanal eine abdichtende Aufnahme einer Hülse sicherstellt.

Aufgabe der Erfindung ist es daher, ausgehend von dem zuletzt genannten Stand der Technik, ein Instrument zu schaffen, mit dem sich ein definierter Gewebekanal unter minimaler Kraftaufwendung und größtmöglicher Sicherheit herstellen läßt.

Gelöst wird diese Aufgabe mit einem Instrument, das die kennzeichnenden Merkmale des Anspruches 1 besitzt.

Kern der Erfindung ist, daß die Messerklinge durch einen in Achsrichtung überstehenden Teilbereich der distalen Rohrkante des Schaftrohres gebildet ist. Die Messerklinge weist dabei einen in Umfangsrichtung des Schaftrohres schneidenden Schliff auf. Weiterhin ist in dem Schaftrohr ein in dem Schaftrohr anordenbarer Einsatz vorgesehen, dessen distales Ende stumpf ausgebildet ist und das sich in etwa auf Höhe des distalen Endes des Schaftrohres befindet. Das distale Ende der Klinge steht dabei über den Einsatz vor.

Die Funktionsweise des Instrumentes bzw. das Zusammenwirken der einzelnen im Anspruch 1 angegebenen Merkmale soll im folgenden erläutert werden. Zur Herstellung eines Zugangs zum Bauchraum wird das erfindungsgemäße Instrument mit der Klinge auf der Bauchdecke angesetzt und dann mit alternierender Drehrichtung abwechselnd um seine Längsachse verdreht. Dabei schneidet die Messerklinge über einen definierten Winkelbereich in die Bauchdecke ein. Der Winkelbereich soll weniger als 360° betragen, um die Bildung von abgelösten Gewebeteilen zu vermeiden. Der Winkelbereich kann ohne weiteres deutlich weniger als 180° betragen. Das über den restlichen Winkelbereich nicht gelöste Gewebe wird während des Eindringens des Instrumentes von dem distalen Ende des Einsatzes ohne Probleme seitlich abgedrängt und zu einem gut abdichtenden Kanal ausgeformt.

Das erfindungsgemäße Instrument erlaubt damit in einfacher Weise unter minimaler Kraftanwendung ein kontrolliertes Eindringen in die Bauchdecke. Die von der Klinge erzeugte glatte Schnittfläche begrenzt zusammen mit dem von dem Einsatz verdrängten Gewebe einen definierten Kanal.

Stößt man während des Eindringens in ein Gewebe auf ein Hindernis, das umgangen werden muß, dann kann das distale Ende des erfindungsgemäßen Instruments durch Drehung um die Klingenspitze um einen Schaftdurchmesser seitlich versetzt werden. Auf diese Weise kann man das erfindungsgemäße Instrument auch nach Eindringen in ein Gewebe in begrenztem Umfang lenken. Die Größe der möglichen Positionsänderung hängt von der Nachgiebigkeit der bereits durchdrungenen Gewebeschichten ab.

Soll mit der erfindungsgemäßen Vorrichtung ein Kanal in einem Gewebe erzeugt werden, das auch stumpf durchdrungen werden kann, so muß nicht unbedingt rotierend geschnitten werden. Das Instrument kann vielmehr einfach eingeschoben werden, wobei die Klinge als Führungshilfe ein seitliches Weglaufen verhindert. Es können also abwechslende Gewebeschichten bedarfsweise mit oder ohne Schnitt durchdrungen werden.

Das erfindungsgemäße Instrument eignet sich daher nicht nur zur Schaffung von z. B. Öffnungen in der Bauchdecke, sondern kann generell überall dort eingesetzt werden, wo ein Gewebekanal geschaffen werden soll.

Vorteilhafte Ausgestaltungen der Erfindung sind in den Unteransprüchen geschützt.

Gemäß Anspruch 2 kann die Klinge zum Beispiel in ihrem distalen Ende spitz zulaufend ausgebildete sein. Eine derartige Klinge läßt sich besonders leicht in einem definierten Punkt auf der Bauchdecke aufsetzen und dort halten. Die Klinge kann jedoch auch ein abgerundetes distales Ende aufweisen. Eine derartige Klingenform ermöglicht besonders schonende Schnitte.

Vorteilhafterweise ist die Klinge gemäß Anspruch 3 ausgebildet und kann in beiden Rotationsrichtungen schneiden, wobei vorteilhaft auch der Spitzenbereich schneidend ausgebildet ist.

Zur seitlichen Verdrängung der geschnittenen Gewebeschichten wird gemäß Anspruch 4 vorteilhafterweise ein Einsatz vorgesehen, dessen Ende eine von der Klingenspitze wegweisende Abschrägung aufweist. Ein derartiger Einsatz drängt das Gewebe in besonders schonender Weise von der Schnittfläche weg.

Wie oben ausgeführt, lassen sich einige Gewebe stumpf durchdringen, d.h. es ist nicht unbedingt der Einsatz eines Schneidwerkzeuges erforderlich. Dieser Tatsache trägt in vorteilhafter Weise eine im Rahmen dieser Erfindung besonders wichtige Ausgestaltung gemäß Anspruch 5 Rechnung. Bei dieser Ausgestaltung lassen sich wahlweise das vorzugsweise abgeschrägte Ende des Einsatzes oder die Klinge in eine distal vorstehende Position bringen. Der Operateur kann durch z.B. einfache Betätigung über einen Handschalter eine der beiden Funktionen wählen. Stößt er z.B. auf Haut, Bindegewebe oder Sehnen, so kommt die Schneidklinge zum Einsatz, ansonsten in weicheren Geweben kann der Einsatz in eine vorgeschobene distale Position gebracht und das Gewebe stumpf durchdrungen werden. Diese Ausgestaltung erlaubt eine besonders schonende, verletzungsarme Erzeugung eines Gewebekanales.

Ist das Schaftrohr mit dem gesamten Instrument drehfest verbunden, so muß durch Drehen des Instrumentes geschnitten werden. Vorteilhaft ist jedoch gemäß Anspruch 6 das Schaftrohr gegenüber dem übrigen Instrument drehbar angeordnet, so daß drehend geschnitten werden kann, ohne daß die übrigen Teile des Instrumentes mitgedreht werden müssen.

Für den Fall, daß das erfindungsgemäße Instrument im Schneidmodus eingesetzt werden soll, ist es erforderlich, das Schaftrohr um seine Längsachse abwechselnd in entgegengesetzten Drehrichtungen über einen definierten Winkelbereich zu verdrehen wird. Eine manuelle Verdrehung ist für den Operateur jedoch schwierig. Daher sieht eine weitere vorteilhafte Ausgestaltung der Erfindung gemäß Anspruch 7 vor, daß an dem Instrument eine Antriebsvorrichung vorgesehen ist, die das Schaftrohr abwechselnd in entgegengesetzten Drehrichtungen um seine Längsachse verdreht. In einer weiteren vorteilhaften Ausgestaltung der Erfindung gemäß Anspruch 8 ist es möglich, daß die Antriebsvorrichtung auf unterschiedliche vorherbestimmte Winkelbereiche einstellbar ist, über die die Verdrehung der Schaftachse und damit der Gewebeschnitt ausgeführt werden soll. Als Antriebseinheit können z.B. ein Elektromotor, aber auch ein pneumatischer Antrieb vorgesehen werden.

Vorteilhaft an den vorher beschriebenen Ausgestaltungen ist, daß der Operateur die Schneidfunktion des Instrumentes durch einfache Aktivierung der Antriebseinrichtung veranlassen kann und seine gesamte Konzentration auf die Lenkung des Instrumentes durch das Gewebe richten kann. Die Aktivierung bzw. Deaktivierung der Antriebseinrichtung kann gemäß Anspruch 9 in besonders einfacher Weise bei einem Instrument wie in Anspruch 5 beschrieben (bei dem wahlweise das stumpfe Ende des Einsatzes oder die Klinge in eine distal überstehende Position bringbar sind) an die Betätigung der Längsverschiebung gekoppelt sein. Bei Vorschieben der Klinge in die Schneidposition wird die Antriebseinrichtung aktiviert. Umgekehrt wird die Antriebsvorrichtung gestoppt, wenn der Einsatz vorgeschoben wird.

Die bislang beschriebenen Ausgestaltungen der Erfindung betreffen ein Instrument, mit dem man unter minimaler Kraftanwendung in ein Gewebe eindringen kann, dieses Eindringen geschieht jedoch blind. Demgegenüber betrifft eine weitere, besonders vorteilhalte Ausgestaltung gemäß Anspruch 10 ein Instrument, bei dem ein mit einer Optik versehener Einsatz in den Schaft eingesetzt ist. Bei Verwendung dieser Ausgestaltung läßt sich der Weg des Instrumentes durch die Gewebeschichten optisch kontrollieren. Die Lichtleiter der Optik durchscheinen die Gewebestrukturen. Eventuelle im Weg des Instrumentes liegende Gefäße können ohne Probleme erkannt und umgangen werden. Insbesondere der Durchtritt durch die Bauchdecke kann so gewählt werden, daß keine darunterliegenden Organe verletzt werden. Da das Peritonaeum transluzent ist, lassen sich bereits vor Durchdringung Verwachsungen erkennen. Das Instrument kann dann unter optischer Kontrolle in einer verwachsungsfreien Zone in die Bauchhöhle gelenkt werden.

Vor dem Objektiv der Optik kann ein Abstandshalter, z.B. ein Glasblock, vorgesehen sein, der das vor dem Instrument befindliche Gewebe auf einer eventuell erforderlichen Abbildungsdistanz hält.

Besonders vorteilhaft ist jedoch, wenn, wie in Anspruch 11 vorgeschlagen, eine Optik eingesetzt wird, die eine Scharfabbildung auch bei Kontakt des Gewebes mit der Frontfläche des Objektives erlaubt. Derartige Optiken erlauben eine besonders deutliche Abbildung mit geringstmöglichem Lichtverlust.

Insbesondere bei der letztgenannten Ausgestaltung kann eine optische Kontrolle auch dann ohne Probleme erfolgen, wenn ein weiches Gewebe mit distal vorgeschobenem Einsatz durchdrungen wird.

In diesem Zusammenhang ist allerdings problematisch, daß gängige Endoskopoptiken in der Regel so beschaffen sind, daß sie nicht direkt auf der Frontfläche des Objektives, sondern im Abstand dazu befindliche Objekte scharf abbilden. Aus Kostengründen besteht ein Interesse daran, derartige gängige Endoskopoptiken auch in Verbindung mit dem erfindungsgemäßen Instrument einzusetzen. Eine weitere vorteilhafte Ausgestaltung der Erfindung sieht daher gemäß Anspruch 12 vor, daß bei Einsatz einer derartigen Standardoptik ein Adapter an das Okular angesetzt wird, der die Brennweite dergestalt verstellt, daß eine Scharfabbildung auf der Frontfläche des Objektives möglich ist.

In einer weiteren, in diesem Zusammenhang vorteilhaften Ausgestaltung gemäß Anspruch 13 ist vorgesehen, daß der Adapter zwischen zwei Brennweiten einstellbar ausgebildet ist. Bei Einstellung der einen Brennweite erfolgt eine Abbildung auf der Frontfläche des Objektives. Bei Einstellung der anderen Brennweite werden Objekte im Abstand zum Objektiv abgebildet. Ein derartiges Instrument läßt sich optimal an die unterschiedlichen Betrachtungserfordernisse anpassen. Während des Durchdringens durch das Gewebe erfolgt die Abbildung direkt vom Objektiv. Nach Durchstoßen der Bauchdecke und Eindringen in die Bauchhöhle kann auf die andere Brennweite umgeschaltet werden, und es lassen sich wie bei der Standardoptik Objekte im Abstand betrachten.

Vorteilhaft lassen sich gemäß Anspruch 14 der Einsatz und das Schaftrohr gegeneinander verdrehen. Es ist dadurch dem Operateur möglich, den Schaft zu seitlich schneidender Bewegung der Klinge zu drehen, ohne den Einsatz zu bewegen. Dies ist insbesondere von Vorteil, wenn der Einsatz mit einer Optik versehen ist, da das Bild dann drehfest stehen bleibt.

Im folgenden soll die Erfindung anhand mehrerer unterschiedlicher Ausführungsbeispiele zeigenden Abbildungen näher erläutert werden.
- Fig. 1: zeigt ein Ausführunsbeispiel des erfindungsgemäßen Instrumentes, bei dem Einsatz und Schaftrohr lateral verschiebbar aneinander gelagert sind und sich die Klinge in vorgeschobener Position befindet.
- Fig. 2: zeigt das Ausführungsbeispiel aus Fig. 1; hier jedoch der Einsatz in distal vorgeschobener Position.
- Fig. 3 und 4: zeigen in einer Teilansicht jeweils Ausführungsbeispiele mit unterschiedlichen Klingenformen.
- Fig. 5: zeigt ein weiteres Ausführungsbeispiel des erfindungsgemäßen Instrumentes, bei dem eine Antriebseinrichtung für das Schaftrohr und ein an dem Okular angesetzter Adapter vorgesehen sind.

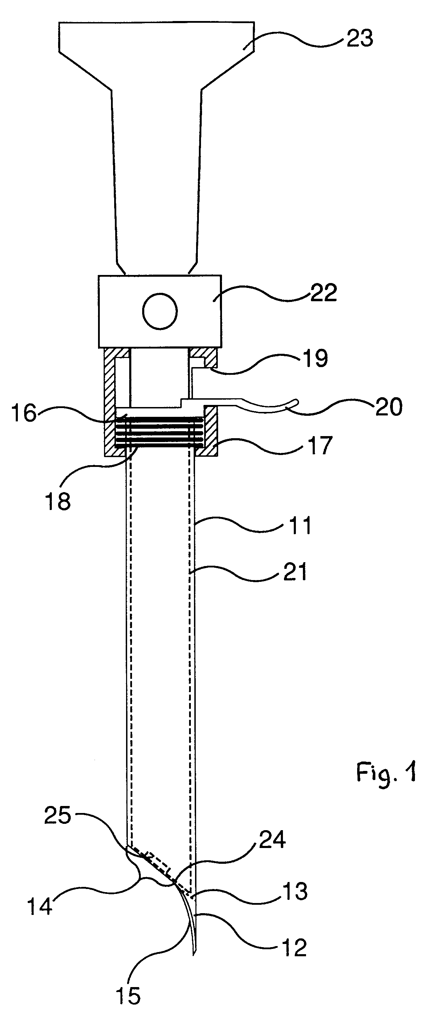

Das in Fig. 1 gezeigte Ausführungsbeispiel der erfindungsgemäßen Vorrichtung besitzt ein Schaftrohr 11, das im Bereich seines distalen Endes eine Klinge 12 aufweist. Die Klinge 12 erstreckt sich in einem Teilbereich des Schaftumfangs, der durch die gestrichelte Linie 13 bezeichnet ist, im wesentlichen in Richtung der Schaftachse. Die Klinge 12 steht dabei deutlich über den verbleibenden Rohrvorderkantenbereich 14 vor. Die gestrichelte Linie 13 bezeichnet die Verlängerung des Rohrvorderkantenbereiches 14 in gerader Schnittfläche nach Art eines üblichen Kanülenendes, über die die Klinge 12 aus der so gebildeten geraden Schnittfläche herausragend ausgebildet ist.

Im Bereich mindestens einer ihrer Seitenkanten 14 besitzt die Klinge 12 einen (hier nicht abgebildeten) Schneidschliff.

An seinem proximalen Ende ist an dem Schaftrohr 11 ein umlaufender Vorsprung 16 vorgesehen, der auf einer in einem Gehäuse 17 abgestützten Druckfeder 18 aufliegt. Zur Verdeutlichung ist das Gehäuse 17 im Schnitt dargestellt.

Die Druckfeder 18 drückt den Vorsprung 16 in Pfeilrichtung. An dem umlaufenden Vorsprung 16 ist weiterhin ein durch eine Öffnung 19 des Gehäuses 17 nach außen führender Hebel 20 vorgesehen. Mit Hilfe des Hebels 20 kann das Schaftrohr 11 gegen die Kraft der Feder 18 bewegt werden.

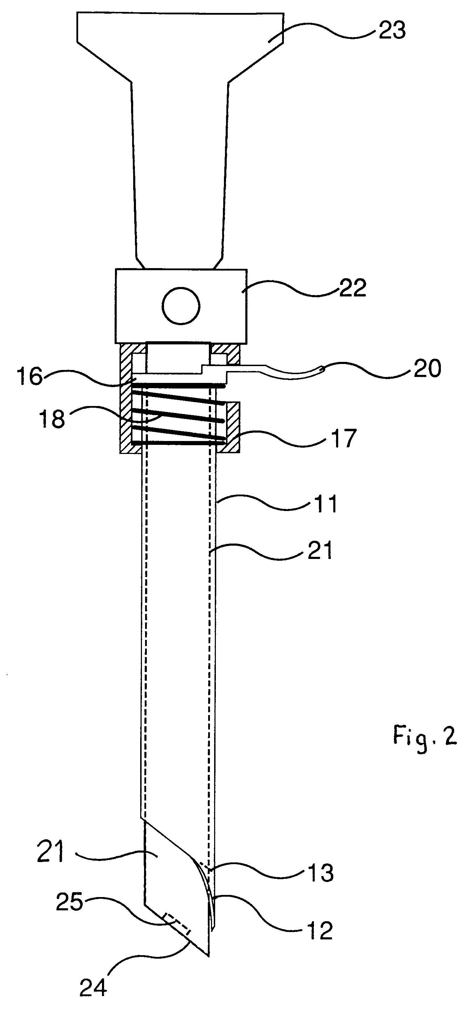

In bevorzugter Ausführungsform sind der Einsatz 21 und das Schaftrohr 11 gegeneinander verdrehbar ausgebildet. Wie in den Fig. 1 und 2 dargestellt, ist dazu die Öffnung 19 des Gehäuses 17 über einen Umfangsbereich ausgebildet, so daß mit Hilfe des Hebels 20 das Schaftrohr 11 nicht nur axial verschoben, sondern auch gedreht werden kann.

In dem Schaftrohr 11 ist ein mit einer Optik versehener Einsatz 21 angeordnet. Der Einsatz 21 ist ortsfest in einer an dem Gehäuse 17 angeordneten Klemmvorrichtung 22 aufgenommen. Im Bereich seines proximalen Endes besitzt der Einsatz 21 ein Okular 23. An seinem distalen Ende 24 ist ein Objektiv 25 vorgesehen. Man erkennt, daß das distale Ende 24 des Einsatzes 21 in Richtung auf das distale Ende der Klinge 12 ansteigend abgeschrägt ist. Auf diese Weise läßt sich, wie oben ausgeführt, eine optimale Verdrängung des von der Klinge 12 bzw. deren Seitenkante 15 angeschnittenen Gewebes erzielen.

Wie in Fig. 1 gezeigt, befindet sich die Klinge 12 gegenüber dem Einsatz 21 in distal vorgeschobener Position. Diese Position läßt sich erreichen, wenn der Hebel 20 gegen den Druck der Feder 18 herabgedrückt gehalten wird.

Möchte der Operateur dagegen mit dem distalen Ende 24 des Einsatzes 21 zuerst (also stumpf)in das Gewebe eindringen, so genügt es, den Hebel 20 loszulassen. Wie in Fig. 2 dargestellt, drückt die Feder 18 dann den Vorsprung 16 mit dem daran befestigten Schaftrohr 11 nach oben. Die Folge ist, daß das distale Ende 24 des Einsatzes 21 sich nun in einer distal vorgeschobenen Position gegenüber der Klinge 12 befindet. Wie oben bereits mehrfach erwähnt, ist es ohne weiteres möglich, mit dem abgeschrägten Ende 24 des Einsatzes 21 in einige Gewebe einzudringen. Insbesondere dann ist es von Vorteil, wenn die in dem Einsatz 21 vorgesehene Optik eine Scharfabbildung an der Frontfläche des Objektives 25 ermöglicht.

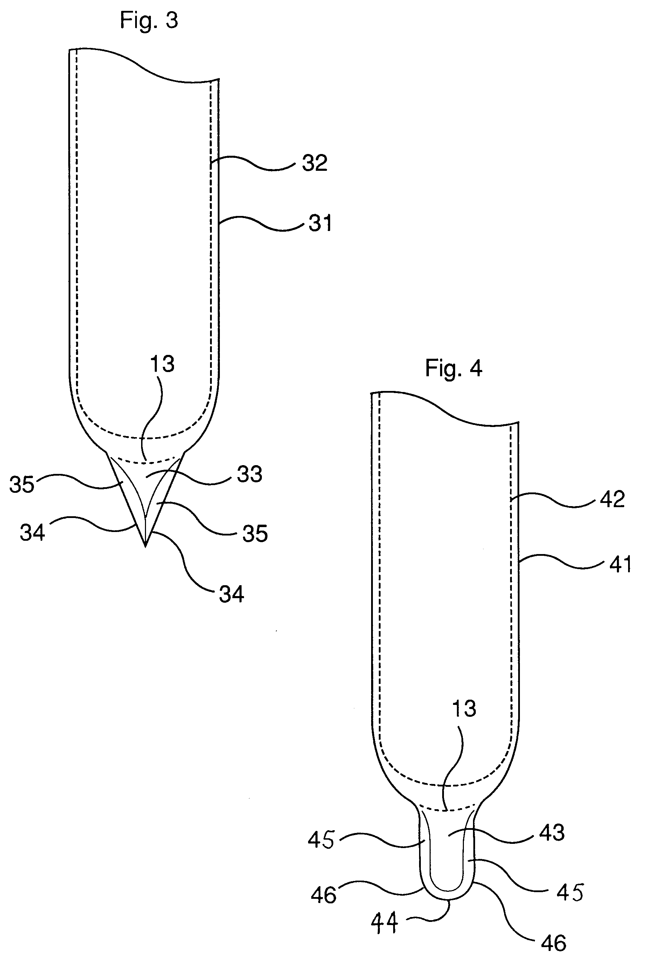

Die Darstellungen in Fig. 3 und 4 zeigen nur den distalen Endbereich von Ausführungsbeispielen der Erfindung, hier allerdings in Seitenansicht.

In Fig. 3 erkennt man ein Schaftrohr 31 mit einem Einsatz 32. Am distalen Ende des Schaftrohres 31 ist eine (nun in seitlicher Aufsicht dargestellte) Klinge 33 vorgesehen, deren beide Seitenkanten 34 jeweils einen Schneidschliff 35 besitzen. Die Klinge 33 ist spitz zulaufend ausgebildet.

Demgegenüber besitzt das in Fig. 4 gezeigte Ausführungsbeispiel ein Schaftrohr 41 (mit Einsatz 42), an dessen distalem Ende eine Klinge 43 vorgesehen ist, deren distales Ende 44 abgerundet ist.

Die Klinge 43 besitzt einen sich über beide Seitenkanten 46 erstreckenden, umlaufenden Schneidschliff 45.

Letztendlich wird es von den Operationsbedingungen abhängen, welche Klingenform und welchen Schliff man wählt. Um diesbezüglich die Einsatzmöglichkeiten der erfindungsgemäßen Vorrichtung zu erweitern, ist es auch denkbar, das Schaftrohr abnehmbar auszugestalten. Dies würde einen einfachen Klingentausch ermöglichen.

Gegenüber den dargestellten Ausführungsbeispielen sind im Rahmen der Erfindung Varianten möglich. Betrachtet man Figur 1, so sieht man, daß die Klinge 12 den Rohrvorderkantenbereich 14 eines Schaftrohres 11 überragend vorgesehen ist, bei dem der Rohrvorderkantenbereich 14 schräg zur Achse des Schaftrohres 11 ausgerichtet ist. Dieser Bereich kann auch eine gebogene Fläche ausbildend geformt sein. In Sonderfällen könnte der Rohrvorderkantenbereich 14 außerhalb der Klinge 12 auch unter stumpferem Winkel zur Achse des Schaftrohres 11 oder quer zu dieser ausgebildet sein.

Wie bei den bekannten Trokarhülsen üblich, könnte das Schaftrohr 11, nach dem der Gewebekanal geschaffen ist, in diesem verbleiben und als Trokarhülse benutzt werden. Zur Verwendung als Laparoskopieport wäre dann eine übliche Ventileinrichtung zusätzlich zu den in den Figuren 1 und 2 dargestellten Teilen vorzusehen, beispielsweise in der dargestellten Klemmvorrichtung 22.

Bei der Verwendung des erfindungsgemäßen Schaftrohres 11 als Laparoskopieport würde aber die Klinge 12 stören, da sie das im Körper liegende Ende des Laparoskopieports traumatisierend gestaltet. Es bestünde Verletzungsgefahr.

Besser ist es daher, das in den Figuren 1 und 2 dargestellte Instrument nur zur Schaffung des Gewebekanales zu verwenden, anschließend zu entfernen und durch einen Laparoskopieport zu ersetzen. Dieser kann während der Schaffung des Gewebekanales über das Schaftrohr 11 geschoben sein, gegebenenfalls unter Zwischenschaltung eines Zwischenrohres mit konischer, also dilatierend wirkender Spitze.

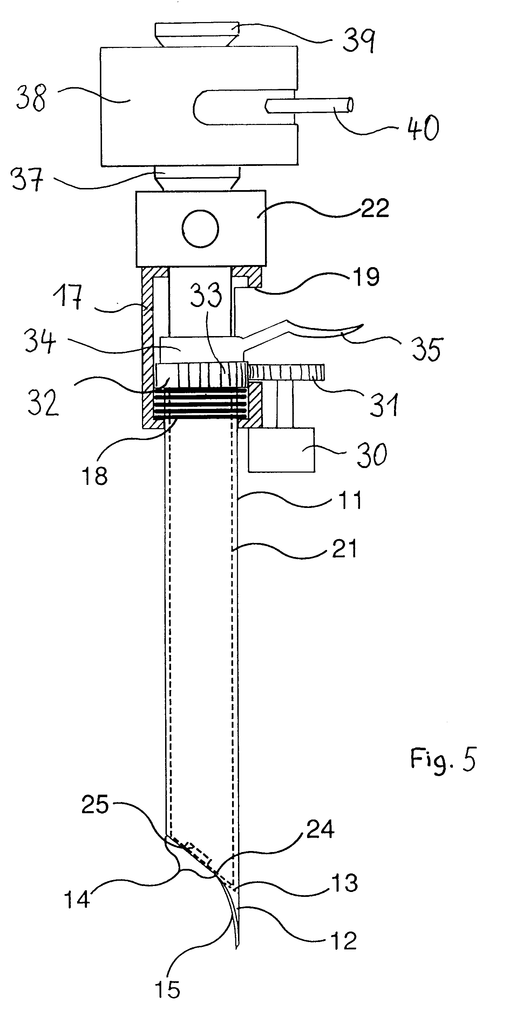

Fig. 5 zeigt schließlich ein Ausführungsbeispiel der Erfindung, das in wesentlichen Merkmalen auf dem in Fig. 1 gezeigten Ausführungsbeispiel aufbaut. Übereinstimmende Merkmale wurden daher mit denselben Bezugszeichen, wie für die Ausführungsform in Fig. 1 verwendet, bezeichnet. Im Unterschied zu Fig. 1 ist bei der hier gezeigten Ausführungsform nun jedoch eine als Elektromotor 30 ausgebildete Antriebseinrichtung an dem Gehäuse 17 angeordnet. Der Elektromotor 30 treibt über ein Ritzel 31 ein fest mit dem Schaftrohr 11 verbundenes Zahnrad 32 an. Der Elektromotor kann zum Zwecke der Erzeugung einer alternierenden Drehbewegung entweder alternierend angesteuert werden oder beispielsweise mit einem Wendegetriebe versehen sein. Man erkennt, daß das Zahnrad 32 nur an einem Teil seines Umfanges mit einer Zahnung 33 versehen ist. Bei dem hier gezeigten Ausführungsbeispiel kann das Schaftrohr 11 damit nur über einen begrenzten Winkelbereich verdreht werden. Selbstverständlich kann auch eine Zahnung über den gesamten Umfang des Zahnrades 32 vorgesehen werden. Eine Begrenzung des Verdrehwinkelbereiches kann dann über eine entsprechende Ansteuerung des Elektromotores vorgesehen werden. Das Zahnrad 32 ist gegen die Feder 18 verschiebbar in dem Gehäuse 17 gelagert. Mittels eines über einen Hebel 35 betätigbaren Gleitring 34 kann das Zahnrad 32 gegen die Feder 18 verschoben werden.

Im gezeigten Fall ist der Gleitring 34 über den Hebel 35 in distaler Richtung bewegt worden und hat dabei das Zahnrad 32 mit seiner Zahnung 33 in Eingriff mit dem Ritzel 31 des Elektromotores 30 gebracht. Wird in dieser Stellung der Hebel 35 losgelassen, so drückt die Feder 18 das Zahnrad 32 und den Gleitring 34 in proximaler Richtung, wodurch die Zahnung 33 und das Ritzel 31 außer Eingriff kommen. So wird auf einfache Weise sichergestellt, daß der Drehantrieb des Schaftrohres 11 nur bei distal gegenüber dem Einsatz 21 vorgeschobener Klinge 12 erfolgt. Sinnvollerweise kann ein Schalter vorgesehen sein, der bei Betätigung des Hebels 35 in distaler Richtung den Elektromotor aktiviert. Auf diese Weise kann der Operateur durch einfaches Verschieben des Hebels 35 in distaler Richtung den Eingriff zwischen Zahnrad 32 und Ritzel 31 herstellen und dabei gleichzeitig den Elektromotor 30 einschalten.

Anstelle eines Elektromotors kann selbstverständlich auch eine andere Form des Antriebes vorgesehen werden. Wichtig ist lediglich, daß die gewählte Antriebseinrichtung problemlos mit dem Instrument verbindbar ist und einen alternierenden Antrieb in entgegengesetzte Drehrichtungen ermöglicht.

Ein weiterer Unterschied zu dem in Fig. 1 gezeigten Ausführungsbeispiel besteht darin, daß das hier gezeigte Instrument an seinem proximalen Ende ein Okular 37 aufweist, an dem ein Adapter 38 angesetzt ist. Der Adapter 38 trägt seinerseits an seinem proximalen Ende ein weiteres Okular 39. Mittels des Adapters 38 läßt sich die Abbildeeigenschaft der in dem Einsatz 21 angeordneten Optik beeinflussen. So ist es z.B. möglich, eine Optik, die im Abstand zu der Linse 25 angeordnete Objekte scharf abbildet, mittels des Adapters 38 in eine Optik zu verändern, die auf der Frontfläche der Linse 25 scharf abbildet. Über einen Einstellhebel 40 kann wahlweise zwischen den beiden Abbildungsmöglichkeiten umgeschaltet werden. Auf diese Weise läßt sich das Instrument besonders gut an die unterschiedlichen Operationssituationen anpassen.

## Patentansprüche

1. Medizinisches Instrument zur Schaffung eines zur Aufnahme einer Hülse bestimmten Gewebekanales, mit einem Schaftrohr, in dessen distalem Endbereich eine Messerklinge angeordnet ist, die zur Erzeugung einer Schnittfläche ausgebildet ist, sowie mit einer Einrichtung zur Umformung der Schnittfläche zum Gewebekanal, **dadurch gekennzeichnet**, daß die Messerklinge (12, 33, 43) durch einen in Achsrichtung überstehenden Teilbereich der distalen Rohrkante des Schaftrohres (11, 31, 41) gebildet ist und mit einem in Umfangsrichtung des Schaftrohres (11, 31, 41) schneidenden Schliff (35, 45) versehen ist, und daß ein in dem Schaftrohr (11, 31, 41) anordenbarer Einsatz (21, 32, 42) vorgesehen ist, dessen distales Ende (21) stumpf ausgebildet ist und sich in etwa auf Höhe des distalen Endes des Schaftrohres (11) befindet.

2. Instrument nach Anspruch 1, **dadurch gekennzeichnet**, daß das distale Ende der Klinge (33, 43) spitz oder abgerundet ausgebildet ist.

3. Instrument nach einem der vorhergehenden Ansprüche, **dadurch gekennzeichnet**, daß der Schliff (35, 45) an beiden Seiten der Klinge (33, 43) ausgebildet ist.

4. Instrument nach einem der vorhergehenden Ansprüche, **dadurch gekennzeichnet**, daß das distale Ende (25) des Einsatzes (21) abgeschrägt ist und der Einsatz (21) so in dem Schaftrohr (11) angeordnet ist, daß sein abgeschrägtes Ende (25) auf die Klinge (12) zulaufend ausgebildet ist.

5. Instrument nach einem der vorhergehenden Ansprüche, **dadurch gekennzeichnet**, daß der Einsatz (21) und das Schaftrohr (11) in Richtung ihrer gemeinsamen Längsachse relativ zueinander verschiebbar ausgebildet sind, dergestalt, daß wahlweise die Klinge (12) oder das distale Ende (24) des Einsatzes (21) in eine distal überstehende Position bringbar sind.

6. Instrument nach einem der vorhergehenden Ansprüche, **dadurch gekennzeichnet**, daß das Schaftrohr (11, 31, 41) am Instrument drehbar gelagert ist.

7. Instrument nach einem der vorhergehenden Ansprüche, **dadurch gekennzeichnet**, daß eine Antriebsvorrichtung (30) vorgesehen ist, die das Schaftrohr (11) um seine Längsachse mit einem vorherbestimmten Drehwinkel und mit abwechselnden Drehrichtungen verdreht.

8. Instrument nach Anspruch 7, **dadurch gekennzeichnet**, daß der Drehwinkel der Antriebsvorrichtung (30) einstellbar ist.

9. Instrument nach den Ansprüchen 6 - 8, **dadurch gekennzeichnet**, daß die Antriebsvorrichtung (30) bei Vorschieben der Klinge (12) automatisch einschaltbar ist.

10. Instrument nach einem der vorhergehende Ansprüche, **dadurch gekennzeichnet**, daß im Einsatz (21) eine Endoskopoptik vorgesehen ist, deren Objektiv (25) am distalen Ende (25) des Einsatzes (21) angeordnet ist.

11. Instrument nach Anspruch 10, **dadurch gekennzeichnet**, daß die Optik eine Scharfabbildung der Frontfläche des Objektives (25) ermöglicht.

12. Instrument nach Anspruch 11, **dadurch gekennzeichnet**, daß eine mit einem proximalen Okular (37) ausgestaltete Standardoptik vorgesehen ist, die im Abstand vor dem Objektiv (25) befindliche Objekte scharf abbildet, und daß ein auf das Okular (37) ansetzbarer Adapter (38) vorgesehen ist, der die Brennweite dergestalt verstellt, daß eine Scharfabbildung der Frontfläche des Objektives (25) möglich ist.

13. Instrument nach Anspruch 12, **dadurch gekennzeichnet**, daß der Adapter (38) zwischen zwei Brennweiten einstellbar ausgestaltet ist, dergestalt, daß zwischen einer Scharfabbildung der Frontfläche des Objektives (25) und aus einem Abstand vor dem Objektiv (25) gewählt werden kann.

14. Instrument nach einem der vorhergehenden Ansprüche, **dadurch gekennzeichnet**, daß der Einsatz (21) und das Schaftrohr (11, 31, 41) gegeneinander um die Schaftachse verdrehbar ausgebildet sind.
